Europäisches Patentamt

European Patent Office

Office européen des brevets

(11) Veröffentlichungsnummer: **0 285 993**
**A2**

(12) **EUROPÄISCHE PATENTANMELDUNG**

(21) Anmeldenummer: 88105133.8

(51) Int. Cl.⁴: **A61M 1/34**

(22) Anmeldetag: 30.03.88

(30) Priorität: 09.04.87 DE 3712044
19.01.88 DE 3801292

(43) Veröffentlichungstag der Anmeldung:
**12.10.88 Patentblatt 88/41**

(84) Benannte Vertragsstaaten:
**AT BE CH DE ES FR GB IT LI NL SE**

(71) Anmelder: **Sartorius GmbH.**
**Weender Landstrasse 94-108**
**D-3400 Göttingen(DE)**

(72) Erfinder: **Lehmann, Hans-Dieter, Dr**
**In der Ganswies 2**
**D-7457 Zimmern-Bisingen(DE)**
Erfinder: **Köhn, Heinz-Gerhard, Dr.**
**Grüner Weg 6**
**D-3402 Dransfeld(DE)**

(74) Vertreter: **Köhler, Rudolf et al**
**c/o Sartorius GmbH Weender Landstrasse**
**94-108**
**D-3400 Göttingen(DE)**

(54) **Filterelement zur Abtrennung von cholesterinhaltigen Präzipitaten aus Blutplasma und durch eine Gehäuse ergänzte Einwegfiltereinheit.**

(57) Für die Anwendung zur Abtrennung von cholesterinhaltigen Präzipitaten aus Blutplasma wird ein mehrschichtig aufgebautes Filtermedium (6) verwendet, das aus mindestens einem offenen, Präzipitat-adsorbierenden Filtermedium (7) und einer die Druckverhältnisse im Filterelement steuernden mikroporösen Membran (9) besteht, wobei die spezifisch das Präzipitat adsorbierende Schicht (7) hydrophob und die mikroporöse Membran (9) hydrophil ist. Zusätzlich ist ein positiv geladenes Adsorptionsmedium (9) auf der dem hydrophoben Adsorptionsmedium (7) abgewandten Seite der mikroporösen Membran (8) vorgesehen. Auf diese Weise wird eine große Adsorptionsfläche für die Präzipitate bzw. für Heparin geschaffen und zugängig gehalten.

## Fig. 1

EP 0 285 993 A2

## Filterelement zur Abtrennung von cholesterinhaltigen Präzipitaten aus Blutplasma und durch ein Gehäuse ergänzte Einwegfiltereinheit

Die Erfindung betrifft ein mehrschichtiges Filterelement für die Abtrennung von cholesterinhaltigen Präzipitaten aus Blutplasma und seine Verwendung bei der dynamischen Filtration bzw. bei der Filtration nach dem Cross-Flow-Prinzip.

Der Gehalt an Low Density Lipoproteinen (LDL) im menschlichem Blutplasma ist positiv, derjenige an High Density Lipoproteinen (HDL) negativ, mit dem das Herzinfarkt-Risiko korreliert. Malchesky et al. (US-PS 4.350.156 bzw. EP-A 0041350) haben deshalb vorgeschlagen, Patienten, die an Hypercholesterämie leiden, mit Hilfe der Plasmapherese zu behandeln. Sie sehen dabei vor, daß die cholesterinhaltigen Blutbestandteile mit Hilfe geeigneter Maßnahmen - z.B. der Zugabe von Heparin - abtrennbar gemacht und durch Filtration aus dem Blutplasma abgetrennt werden.

Eine elegante Methode hierfür haben Seidel et al. angewendet lt. DE-OS 31 35 814. Ihr Verfahren vermag HDL im Plasma zu halten und LDL zusammen mit dem in Herzinfarkt-Risikopatienten pathologisch überhöhten Fibrinogen abzutrennen. Sie nutzen hierfür die Tatsache aus, daß sich HDL und LDL sowohl in ihren Molekulargewichten als auch in ihrem HLB (hydrophil-lipophile Balance) unterscheiden (A.K. J. Koumans, A.P. Wildschut, Nutrition and Atherosclerosis: Some Neglected Aspects, Clin. Cardiol 8/1985), S. 549.

| | HDL | LDL |
|---|---|---|
| **Molekulargewicht (Dalton)** | ca. 400.000 | ca. 2 - 3,5 Mio |
| **Cholesterinanteil (%)** | | |
| **d.h. hydrophob = lipophil** | 25 | 47 |
| **Protein (%)** ⎫ | 22 ⎫ | 25 ⎫ |
| ⎬ hydrophil | ⎬ 75 | ⎬ 53 |
| **Lecithin (%)** ⎭ | 53 ⎭ | 28 ⎭ |

Bei diesem Verfahren wird das durch Plasmapherese gewonnene Blutplasma verdünnt und im pH-Wert abgesenkt. Dadurch erhalten die Lipoproteine wie das Fibrinogen positive Überschuß-Ladungen, die mit dem im Puffer zugegebenen Heparin als Polyanion komplexieren. LDL fällt als Komplex mit hohem Hydrophob-Anteil aus, dessen schmalzige Beschaffenheit ein Abfiltrieren unerfreulich gestaltet. Aus diesem Grund schlagen Roßkopf et al. in DE-OS 3310727 zu seiner Abtrennung eine drucklose Cross-Flow-Filtration mit Hilfe einer großflächigen Filterkerze vor, in welcher eine Polycarbonatmembran verwendet wird. Für diesen Filter werden im Anwendungsfall fast zwei m² Membran verwendet, weil ein Großteil der Fläche durch das Präzipitat blockiert wird.

Aus medizinischer Sicht ist das mit dieser Filterausführung verbundene große Totvolumen ein Nachteil. Trotz Verdünnung des Plasmas mit Acetatpuffer wird dem Körper ein großes Plasmavolumen entzogen, welches am Ende der Behandlung möglichst weitgehend zurückzugeben ist.

Eine zwingende Erfordernis des beschriebenen Verfahrens ist die Notwendigkeit, dem Blutplasma wieder den Überschuß an Heparin zu entziehen, in welchem ausgefällt wurde. Es wird bei den Verfahren nach DE-OS 3135814, Seidel et al., in einer Adsorptionskolonne an einen Anion-Austauscher festgehalten. Anschließend wird das gereinigte Plasma - in pH und Volumen korrigiert - zusammen mit den zellulären Blutbestandteilen dem Patienten zurückgegeben.

Der Erfindung liegt daher die Gestaltung eines effizienten Filters zur Abtrennung der Heparin-Komplexe der pathologischen Plasmabestandteile zugrunde. Eine große Leistungsdichte, d.h. kleines Volumen sollte das extracorporale Plasmavolumen minimieren und trotzdem die erforderlichen Kapazitäten schaffen. Ein zusätzliches Ziel ist es, in dem vielstufigen Verfahren nach dem Stand der Technik z.B. nach DE-OS 31 35 814 die Präzipitat-Abtrennung und die Abtrennung des Heparin-Überschusses in einem Vorgang zu vereinen.

Erfindungsgemäß wird dieses Ziel nach den Merkmalen des Hauptanspruches dadurch erreicht, daß der Trennmembran Adsorptionsflächen vor-und gegebenenfalls nachgeschaltet werden. Diese großflächigen

2

Adsorptionsflächen für die Präzipitate bzw. für Heparin sind möglichst frei zugänglich. Bei großer Adsorptionskapazität gewährleisten sie einen drucklosen Durchgang der zu filtrierenden Flüssigkeit auch dann noch, wenn sie bereits weitgehend mit den zu adsorbierenden Materialien belegt sind. Die Druckverhältnisse in diesem Filter werden durch die zwischen die Adsorptionsflächen gelegten mikroporösen Membranen bestimmt. Dadurch wird erfindungsgemäß eine Kanalbildung durch das offene Adsorbens und damit Präzipitat-Durchbrüche verhindert.

Erfindungsgemäß ist die Kombination in dem mehrschichtigen Adsorptions-Filtrations-Verbund so ausgestaltet, daß mit dem Plasma samt Präzipitat ein hydrophobes Vlies kontaktiert wird. Als nächste Schicht folgt eine möglichst hydrophile mikroporöse Membran, anschließend eine Adsorptionsschicht mit positiven Ladungen. Dies kann z.B. eine Membran aus DEAE-Cellulose oder ein Vlies mit DEAE-Gruppen (z.B. DEAE-Papier der Firma Serva) sein oder andere positiv geladene Membranen, wie sie z.B. von der Firma Pall unter Bezeichnung Posidyne oder AMF-Cuno unter Bezeichnung Zetaplus auf den Markt gebracht werden. Eine besonders bevorzugte Ausführungsform ist ein Endlosfaden-Vlies mit positiven Ladungen, wie es - in Analogie zur Ausrüstung von Membranen in den Patentschriften der Firma Pall und AMF-Cuno - aus ungeladenen Endlosfaden-Vliesen hergestellt werden kann.

Filter mit den erfindungsgemäßen mehrschichtigen Filtermedien können in bekannter Weise in Form von plissierten Filtern (z.B. wie bei einem Filter nach der DE-OS 33 44 374 der Firma Sartorius) oder in anderen, die Cross-Flow-Anwendung ermöglichenden Konfigurationen hergestellt werden. Bevorzugt werden Ausführungsformen mit hoher Leistungsdichte, d.h. mit kleinem Totvolumen.

Ein Filter, welches in seinem äußeren Aufbau einem gefalteten Filterelement lt. DE-OS 33 44 374 der Firma Sartorius GmbH entspricht, besteht aus einer gefalteten (plissierten) Membran zwischen zwei Stützvliesen. Durch Abdichtung zwischen den Filtrationsschichten und dem Gehäuse mit Hilfe von Vergußmasse (z.B. Polyurethan) ist das Gerät in zwei Kammern geteilt. Durch die 1. Kammer wird das Plasma rezirkuliert, in welchem das Präzipitat ausgefällt wurde.

Die Vliese des Filters sind hydrophobe Endlosfaden-Vliese aus Polyester mit einem Flächengewicht von ca. 50 g/m$^2$ und einer Fadenstärke von ca. 2 detex. Die mikroporöse Membran als folgende Schicht besteht aus einer Polyamid 6.6-Membran mit den Porengrößen von 0,45 bzw. 0,8 µm. Bei einer Membranfläche von 0,2 m$^2$ betrug die präzipitatseitig installierte hydrophobe Adsorptionsfläche 3,5 m$^2$.

In outdated Humanplasma wurde nach DE-OS 31 35 814 mit Acetat-Puffer und Heparin ein Präzipitat ausgefällt. Das Plasma mit dem gelblich-milchigen Niederschlag wurde durch die 1. Kammer des Filters rezirkuliert. Der eingangsseitige Druck am Filter war 0,04 bar. Im Beispiel 1 (Porenweite 0,45 µm) reichte eine hydrostatische Druckdifferenz von ca. 50 cm Wassersäule zur Filtration aus, in Beispiel 2 (Porenweite 0,8 µm) leerte sich das System bereits bei einer Höhendifferenz von 5 - 10 cm. Das gereinigte Plasma war klar und schwach gelblich.

Die Ergebnisse sind in Tabelle 1 zusammengestellt.

| Tab.1 | Probe Nr. | Cholesterin (mg/dl) | Triglyceride (mg/dl) | Gesamtprotein (g/dl) | Albumin -- (g/dl) |
|---|---|---|---|---|---|
| Ausgangsprobe | 0 | 150 | 70 | 6,0 | 3,8 |
| Filtrat aus Filter mit 0,8 µm | 1 (Anfang) | 34 | 18 | 5,0 | 2,8 |
| | 2 (15 Min) | 32 | 16 | 4,8 | 2,6 |
| | 3 (Schluß) | 32 | 16 | 4,6 | 2,6 |
| Filtrat aus Filter mit 0,45 µm | 1B (Anfang) | 48 | 26 | 6,0 | 3,6 |
| | 2B (15 Min) | 46 | 26 | 6,0 | 3,4 |
| | 3B (Schluß) | 46 | 26 | 6,0 | 3,4 |

3

In einem weiteren Versuch wurde bei der Filtration Heparin eliminiert:

Die Durchführung erfolgte wie bei Beispiel 2. Hinter der Nylon-Membran wurde ein offenes DEAE-Papier der Firma Serva (Nr. 43062) verwendet. Das klare Plasma erwies sich als frei von Heparin.

Der Erfindungsgedanke ist in verschiedenen Ausführungsformen realisierbar. Die nach dem Stand der Technik bekannten Möglichkeiten Filterelemente in verschiedener Anordnung in den verschiedensten Gehäusen unterzubringen, wird daher in der nachfolgenden schematischen Darstellung erläutert. Dabei zeigt:

Fig. 1 einen schematischen Schnitt durch ein Gehäuse mit eingelagertem Filterelement,

Fig. 2 einen schematischen Querschnitt durch das erfindungsgemäße Filterelement.

Fig. 3 schematisch einen Querschnitt durch das Filterelement mit Gehäuse nach der Schnittlinie 3-3 in Fig. 4,

Fig. 4 einen Längsschnitt durch die Einheit nach der Schnittlinie 4-4 in Fig. 3,

Fig. 5 einen Querschnitt durch ein aus drei Faltenblöcken aufgebautes, im ringförmigen Gehäuseteil vormontiertes Filterelement,

Fig. 6 eine abgewandelte Ausführungsform des Gehäuseaufbaues,

Fig. 7 ein Gehäuseende mit abgewandelten Fluidanschlüssen in Seitenansicht, teilweise geschnitten einer bevorzugten Ausführungsform,

Fig. 8 eine Stirnansicht dazu,

Fig. 9 einen Detailschnitt durch die Gehäuseverbindung an den Längsseiten unter Einschluß der Endfalten der Filterelemente und

Fig. 10 eine Detailvergrößerung gemäß dem Ausschnitt 10 in Fig. 9.

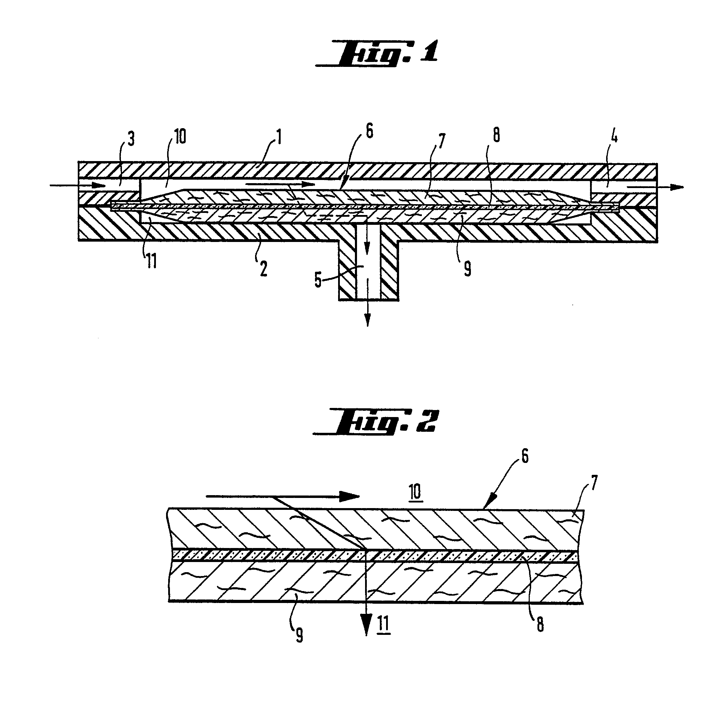

Gemäß Fig. 1 ist das Filterelement 6 mit seinen Rändern dichtend zwischen den beiden Filtergehäuseteilen 1 und 2 eingeklemmt.

Das Filterelement 6 trennt im Retentatraum 10 des Filtergehäuses mit zwei Anschlüssen 3 und 4 vom Permeatraum 11 mit einem Permeatauslaß 5.

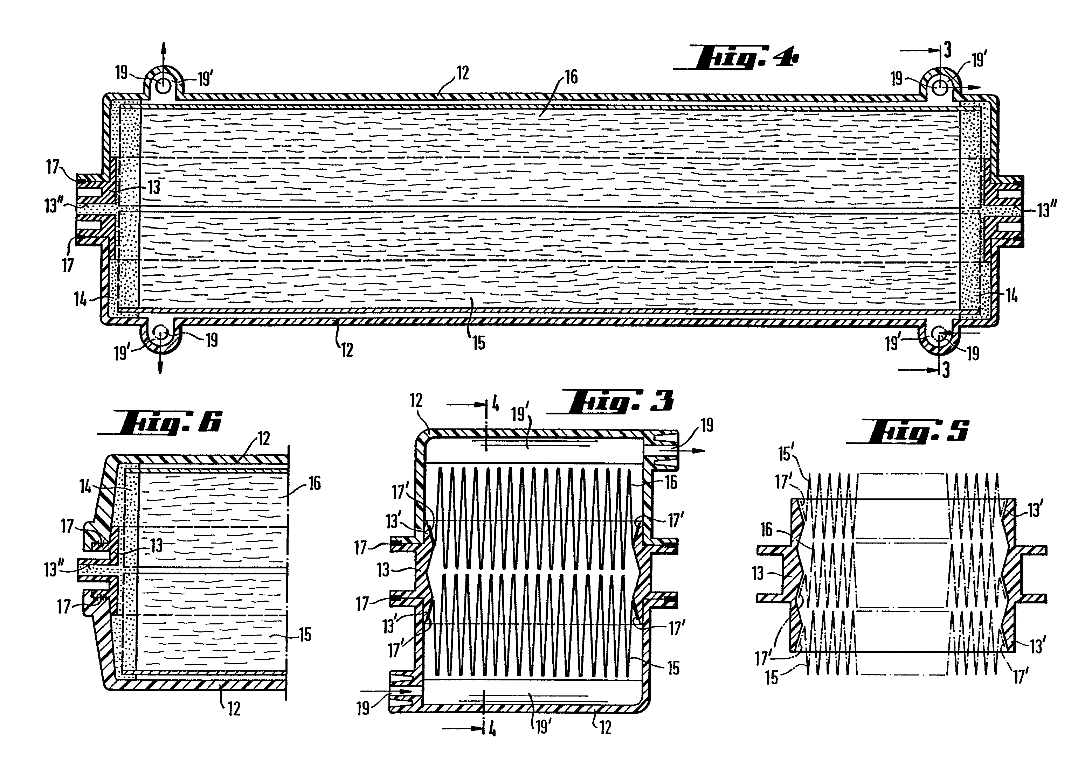

Gemäß Fig. 2 besteht das Filterelement 6 aus einem hydrophoben Vlies 7 mit Präzipitat adsorbierenden Eigenschaften, einer stromabwärts nachfolgenden mikroporösen und hydrophilen Membran 8, welche die Druckverhältnisse im Filterelement steuert und einem vorzugsweise positiv geladenen hydrophilen Stützvlies 9 mit drainierender Wirkung. Das zu behandelnde Blutplasma rezirkuliert in der Retentatkammer 10 und überströmt nach dem Cross-Flow-Prinzip das Filterelement 6 durch die Fluidanschlüsse 3 und 4. Das Präzipitat wird über den Permeatauslaß 5 abgeführt.

Die nachfolgend beschriebenen praktischen Ausführungsbeispiele zeigen Filtereinheiten mit großer Filterfläche bzw. Adsorptionsfläche auf engem Raum, großer Packungsdichte und sehr geringem Totvolumen.

Gemäß Fig. 3 und 4 besteht die Filtereinheit aus den beiden identischen Gehäuseschalen 12 mit Anschlüssen 19, die in Gehäusequerrichtung verlaufende Flüssigkeitsverteiler 19' übergehen.

Zwischen beiden Gehäuseschalen 12 ist ein ringförmiges Gehäuseteil 13 angeordnet, welches mit Dichtflanschen ausgestattet und mit entsprechenden Dichtflanschen der Gehäuseschalen 12 dichtend verbunden ist, wobei die Dichtung 17 entweder durch Ultraschallschweißung oder durch Verklebung erzeugt werden kann. In der unteren Gehäuse schale 12 ist in plissierter Form das Präzipitat adsorbierende hydrophobe Filterelement 15 angeordnet, welches mit seinen Faltenkanten in Längsrichtung des Gehäuses 12,13 verläuft und dessen Endfalten 17' an Dichtlippen 13' des ringförmigen Gehäuseteils 13 dichtend anliegen. In gleicher Weise ist in Strömungsrichtung nachgeschaltet ein plissiertes Membranfilterelement 16, welches das eigentliche zu gewinnende Permeat hindurchläßt. Die Stirnflächen der beiden Filterpakete 15,16 sind im Gehäuse 12,13 durch Vergußmasse 14 abgedichtet, die durch Gehäuseöffnungen 13'' z.B. im mittleren Gehäuseteil 13 eingebracht werden kann.

Das zu filtrierende Medium strömt in die untere Gehäuseschale 12 in den einen Anschluß 19 ein, überströmt die in Längsrichtung verlaufenden Falten des adsorbierenden Filterelementes 15 und verläßt die untere Gehäuseschale durch den anderen Auslaß 19, wobei die Überströmung nach dem Cross-Flow-Prinzip erfolgt. Die vom Filterelement 15 nicht adsorbierten Bestandteile der Flüssigkeit durchdringen das Filterelement 15 und durchdringen ihrerseits nach dem statischen Filtrationsprinzip das eigentliche Membranfilterelement 16, wobei das Permeat aus dem einen oder anderen oder aus beiden Anschlüssen 19 der oberen Gehäuseschale abgezogen werden kann.

Bei der Ausführungsform nach Fig. 5 ist dem eigentlichen Membranfilterelement 16 ein weiteres Filterelement 15' ebenfalls in plissierter Form mit adsorbierenden Eigenschaften nachgeschaltet. Ohne daß die Gehäuseabmessungen und die Formen abgeändert werden müssen, erfolgt die Unterbringung von drei Filterelementen durch Verringerung der Plissierhöhe der einzelnen Falten.

Fig. 6 zeigt einen Detailpunkt bezüglich der Verbindung des ringförmigen Gehäuseteiles 13 mit den beiden schalenförmigen Gehäuseteilen 12 und die Anordnung von Öffnungen 13" zum Einspritzen einer fließfähigen und aushärtbaren Vergußmasse 14 für die Stirnseiten der Faltenpakete 15,15',16 bzw. auch für die mögliche Einbringung von Vergußmasse 14 für die Abdichtung der Endfalten in Längsrichtung.

Die Anordnung eines ringförmigen Gehäuseteiles 13 zwischen zwei identischen schalenförmigen Gehäuseteilen 12 hat den Vorteil, daß gegebenenfalls durch unterschiedlich hohe ringförmige Gehäuseteile Filtereinheiten mit unterschiedlich großen Filterflächen geschaffen werden können, indem die Plissierhöhe der Falten verändert wird und die Filtereinheiten aus identischen Gehäuseschalen 12 und unterschiedlich hohen ringförmigen Gehäuseteilen 13 gebildet werden können.

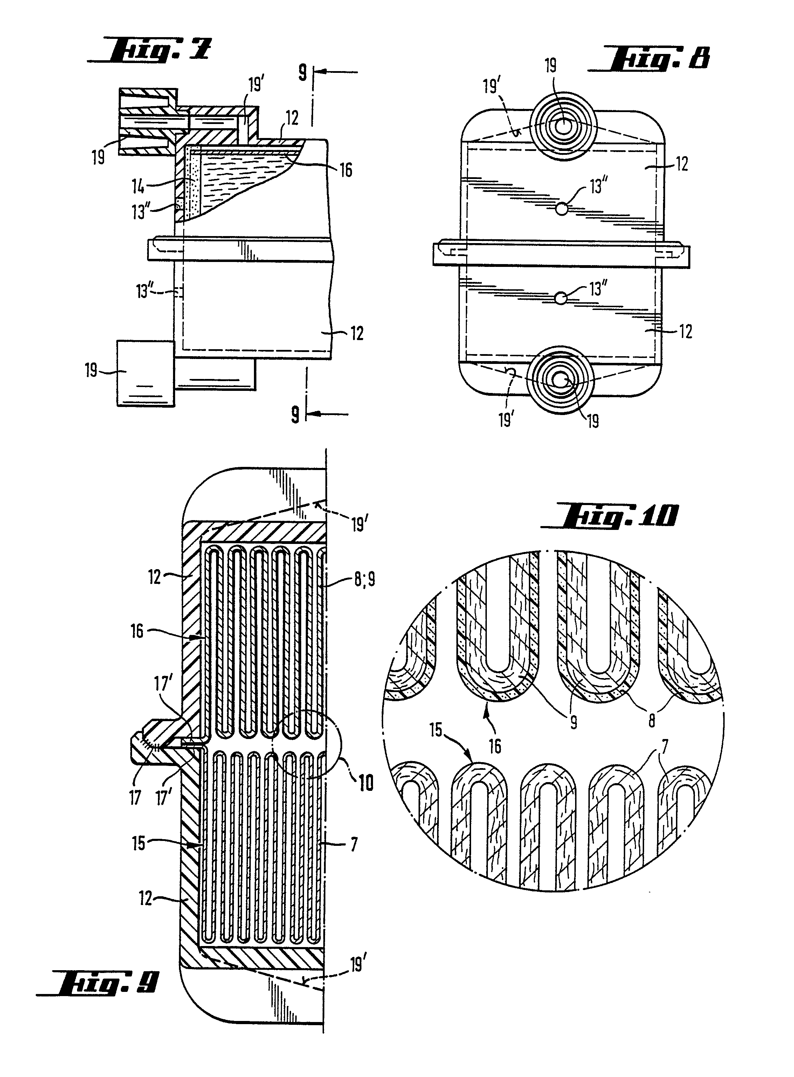

Bei der bevorzugten Ausführungsform nach Fig. 7 bis 9 sind die Anschlüsse 19 an den Stirnseiten angeordnet. Die Gehäuseverbindung 17 an den Längsseiten schließt dabei die Endfalten 17' der beiden Filterelemente 15,16 partikeldicht z.B. durch Klemmung und/oder Ultraschallverschweißung ein.

Das Filterelement 16 besteht hierbei aus der stromaufwärts liegenden mikroporösen Membran 8 und dem Stützvlies 9 gemäß Fig. 2, die gemeinsam zu einem Faltenpaket plissiert sind. Daraus ergibt sich für das stromaufwärts vorgelagerte Filterelemente 15 eine gegenüber dem Filterelement 16 größere Faltenanzahl und Adsorptionsfläche.

Allen Ausführungsbeispielen ist gemeinsam, daß auf engstem Raum große Adsorptions-und Filterflächen mit geringem Totvolumen untergebracht sind. Anders als bei runden Filterkerzen nach dem Stand der Technik mit radial nach außen sich erweiternde Faltenöffnungen, liegen hier die Faltenöffnungen sowohl auf der Retentatseite (Adsorptionsseite) als auch auf der Permeatseite zur Reduzierung des Totvolumens eng aneinander und gehen in kleinvolumige Strömungskammern über.


## Ansprüche

1. In einem Gehäuse (1,2) mit mehreren Fluidanschlüssen (3,4,5) für die dynamische Filtration einschließbares Filterelement (6) zur Abtrennung von cholesterinhaltigen Präzipitaten aus Blutplasma, gekennzeichnet durch ein Präzipitat absorbierendes hydrophobes plissiertes und enge Faltenöffnungen aufweisenden Filtermedium (7) und eine stromabwärts nachfolgende mikroporöse, hydrophile und die Druckverhältnisse im Filterelement steuernde Membran (8), welche gegebenenfalls durch eine drainierende Stützschicht (9) ergänzt ist.

2. Filterelement nach Anspruch 1, gekennzeichnet durch ein zusätzliches, positiv geladenes Adsorptionsmedium (9) auf der dem hydrophoben Adsorptionsmedium (7) abgewandten Seite der mikroporösen Membran (8).

3. Filterelement nach Anspruch 1 und 2, dadurch gekennzeichnet, daß das hydrophobe, Adsorptionsmedium (7) durch ein Polyester-Endlosfaden-Vlies gebildet ist.

4. Filterelement nach Anspruch 1 und 2, dadurch gekennzeichnet, daß die hydrophile mikroporöse Membran (8) durch eine Nylon-Membran gebildet ist.

5. Filterelement nach Anspruch 2, dadurch gekennzeichnet, daß das positiv geladenes Adsorptionsmedium (9) durch eine Membran oder ein Vlies mit Anionen-Austauschereigenschaft gebildet ist.

6. Filterelement nach Anspruch 1 bis 5 dadurch gekennzeichnet, daß das Membranfilterelement (16) aus einem dicht in Falten gelegten Filterblatt mit Drainageschichten gebildet ist und zusammen mit dem plissierten Filtermedium (7) in einem umschließenden Gehäuse (12,13) aus Kunststoff mit diesem zu einer Einwegfiltereinheit vereint ist.

7. Einwegfiltereinheit nach Anspruch 6, dadurch gekennzeichnet, daß das adsorbierende Filterelement (15) und das Membranfilterelement (16) unmittelbar ohne Sammelleitung als Faltenpakete aneinander anschließen.

8. Einwegfiltereinheit nach Anspruch 6 und 7, dadurch gekennzeichnet, daß dem Membranfilterelement (16) ein Filterelement (15') mit Adsorptionseigenschaften nachgeordnet ist.

9. Einwegfiltereinheit nach Anspruch 6 bis 8, dadurch gekennzeichnet, daß das Gehäuse aus mindestens zwei länglichen Gehäuseschalen (12,12) aufgebaut ist und die Faltenkanten der Filterelemente (15,15',16) in Gehäuselängsrichtung verlaufen und die Versorgungsanschlüsse (19) und Entsorgungsanschlüsse (19) in quer über die Faltenkanten verlaufende Verteiler (19') übergehen und die Endfalten der Faltenpakete (15,15',16) an den Längswänden der Gehäuseschalen (12,12,13) dichtend anliegen und die Stirnseiten der Faltenpakete (15,15',16) an den Gehäusestirnseiten (14) abgedichtet sind.

10. Einwegfiltereinheit nach Anspruch 6 bis 9, dadurch gekennzeichnet, daß das Gehäuse aus drei Gehäuseformteilen nämlich einem mittleren ringförmigen Gehäuseteil (13) und zwei Schalenteilen (12) gebildet ist, die die Filterelemente (15,15',16) einschließen und dichtend miteinander verbunden sind.

11. Einwegfiltereinheit nach Anspruch 6 bis 10, dadurch gekennzeichnet, daß die Gehäuseteile (12,13) aus Kunststoff gebildet sind und diese und die Filterelemente (15,15',16) unlösbar verbunden sind.

12. Einwegfiltereinheit nach Anspruch 6 bis 11, dadurch gekennzeichnet, daß die Gehäuseteile (12,13) durch Verschweißung verbunden sind.

13. Einwegfiltereinheit nach Anspruch 6 bis 11, dadurch gekennzeichnet, daß die Endfalten (17') der Faltenpakete (15,15',16) durch Preßdichtungen (7) des ringförmigen Gehäuseteiles (13) abgedichtet sind.

14. Einwegfiltereinheit nach Anspruch 6 bis 13, dadurch gekennzeichnet, daß die Stirnflächen der Faltenpakete (15,15',16) durch in das Gehäuse (12,13) einspritzbare Kunststoffmasse (14) abgedichtet sind.

15. Einwegfiltereinheit nach Anspruch 6 bis 14, dadurch gekennzeichnet, daß die Endfalten (17') der Filterelemente (15,16) in der längsseitigen Gehäuseverbindung (17) partikeldicht eingeschlossen sind.

16. Einwegfiltereinheit nach Anspruch 15, dadurch gekennzeichnet, daß die Endfalten (17') zwischen den Längsflanschen der Gehäuseteile (12) durch Klemmung dichtend gehalten und die Gehäuseteile (12) durch Ultraschallschweißung verbunden sind.

## Fig. 1

## Fig. 2

*Fig: 7*

*Fig: 8*

*Fig: 10*

*Fig: 9*